# EUROPÄISCHE PATENTANMELDUNG

(11) **EP 0 887 368 A1**
(43) Veröffentlichungstag der Anmeldung: **30.12.1998**
(21) Anmeldenummer: 98108163.1
(22) Anmeldetag: 05.05.1998
(51) Int. Cl.: C08G 81/02, C08G 18/62, C09D 187/00

(54) **Blutverträgliches und bakterienabweisendes Blockcopolymer**

(30) Priorität: 24.06.1997 DE 19726735
(71) Anmelder: HÜLS AKTIENGESELLSCHAFT, 45764 Marl (DE)
(72) Erfinder: Anders, Christine, Dr., 45721 Haltern (DE); Lorenz, Günter, Dr., 52068 Aachen (DE)

(57) **Zusammenfassung**

Die Erfindung betrifft ein Blockcopolymer, enthaltend Sulfonat- und Carboxylgruppen tragende Blöcke A und Polyurethan- oder Polyesterblöcke B, sowie Compounds aus diesem Blockcopolymer und einem weiteren Polymer. Die Erfindung betrifft weiter ein Verfahren zur Herstellung des Blockcopolymers, bei dem man ein Copolymer C, das Hydroxylgruppen und/oder Aminogruppen sowie mindestens ein sulfonatgruppenhaltiges Monomer (a) und mindestens ein carboxlgruppenhaltiges Monomer (b) enthält, mit einem Polyurethan D mit endständigen Isocyanatgruppen oder einem Polyester D mit endständigen Carbonsäurechloridgruppen umsetzt. Die Erfindung betrifft auch Erzeugnisse, die ganz oder teilweise mit einem solchen Blockcopolymer beschichtet wurden oder ganz oder teilweise aus einem solchen Compound bestehen, für medizinische, hygienische, technische, nahrungsmittel- oder biotechnische Zwecke.

## Beschreibung

Die Erfindung betrifft ein blutverträgliches und bakterienabweisendes Blockcopolymer, das Sulfonat- und Carboxylgruppen tragende Blöcke sowie Polyurethan- oder Polyesterblöcke enthält. Die Erfindung betrifft weiter ein Verfahren zur Herstellung des Blockcopolymers sowie dessen Verwendung. Die Erfindung betrifft schließlich Erzeugnisse, die ganz oder teilweise mit dem erfindungsgemäßen Blockcopolymer beschichtet sind.

### 1. Stand der Technik

Die Ansiedelung und Vermehrung von Bakterien auf Oberflächen ist eine in der Regel unerwünschte Erscheinung, die häufig mit nachteiligen Folgen verbunden ist. So können in der Trinkwasser- und Getränketechnik Bakterienpopulationen zu einer gesundheitsgefährdenden Qualitätsminderung führen. Bakterien auf oder in Verpackungen bewirken häufig den Verderb von Lebensmitteln oder verursachen sogar Infektionen bei dem Verbraucher. In steril zu betreibenden biotechnischen Anlagen stellen systemfremde Bakterien ein erhebliches prozeßtechnisches Risiko dar. Solche Bakterien können mit Rohstoffen eingetragen werden oder bei mangelhafter Sterilisation in allen Anlageteilen zurückbleiben. Teile der Bakterienpopulation können sich durch Adhäsion dem normalen Flüssigkeitsaustausch beim Spülen und Reinigen entziehen und sich im System vermehren.

Weiterhin sind Bakterienansiedelungen in Wasseraufbereitungsanlagen (z.B. zur Entsalzung durch Membranen) oder auch in Behältern bekannt, die mit gelösten oder flüssigen unverdünnten organischen Substanzen gefüllt sind und für Bakterienpopulationen vorteilhafte Bedingungen aufweisen. Solche mikrobiellen Belegungen können in erheblichem Umfang zur Blockierung und/oder korrosiven Zerstörung der Anlage führen.

Besondere Bedeutung kommt dem Schutz vor Bakterienanhaftung und -ausbreitung in der Ernährung, der Pflege, hier insbesondere in der Altenpflege, und in der Medizin zu. Bei Massenbeköstigungen oder -ausschank existieren besonders dann erhebliche Risiken, wenn zur Vermeidung von Abfall von Einweggeschirr abgesehen wird und eine nur unzureichende Reinigung des Mehrweggeschirrs erfolgt. Die schädliche Ausbreitung von Bakterien in lebensmittelführenden Schläuchen und Rohren ist ebenso bekannt wie die Vermehrung in Lagerbehältern sowie in Textilien in feuchter und warmer Umgebung, z.B. in Bädern. Solche Einrichtungen sind bevorzugte Lebensräume für Bakterien, ebenso wie bestimmte Oberflächen in Bereichen mit hohem Publikumsverkehr, so z.B. in öffentlichen Verkehrsmitteln, Krankenhäusern, Telefonzellen, Schulen und insbesondere in öffentlichen Toiletten.

In der Alten- und Krankenpflege erfordern die häufig geminderten Abwehrkräfte der Betroffenen sorgfältige Maßnahmen gegen Infektionen, insbesondere auf Intensivstationen und in der häuslichen Pflege.

Besondere Sorgfalt bedarf die Verwendung medizinischer Gegenstände und Geräte bei medizinischen Untersuchungen, Behandlungen und Eingriffen, vor allem dann, wenn derartige Geräte oder Gegenstände mit lebendem Gewebe oder mit Körperflüssigkeiten in Kontakt kommen. Im Falle von Langzeit- oder Dauerkontakten, beispielsweise bei Implantaten, Kathetern, Stents, Herzklappen und Herzschrittmachern, können Bakterienkontaminationen zu einem lebensbedrohenden Risiko für den Patienten werden.

Es wurde bereits auf vielfältige Weise versucht, die Ansiedelung und Ausbreitung von Bakterien auf Oberflächen zu unterbinden. In J. Microbiol. Chemoth. **31** (1993), 261-271 beschreiben S.E.Tebbs und T.S.J.Elliott lackartige Beschichtungen mit quaternären Ammoniumsalzen als antimikrobiell wirkenden Komponenten. Es ist bekannt, daß diese Salze von Wasser, wäßrigen oder anderen polaren Medien sowie von Körperflüssigkeiten aus dem Beschichtungsmaterial herausgelöst werden und ihre Wirkung somit nur von kurzer Dauer ist. Dies gilt gleichermaßen für die Einarbeitung von Silbersalzen in Beschichtungen, so beschrieben in WO 92/18098.

T. Ouchi und Y. Ohya beschreiben in Progr.Polym.Sci. **20** (1995), 211 ff., die Immobilisierung von bakteriziden Wirkstoffen auf Polymeroberflächen durch kovalente Bindung oder ionische Wechselwirkungen. Häufig sind in solchen Fällen die keimtötenden Wirkungen gegenüber dem reinen Wirkstoff deutlich reduziert. Heteropolare Bindungen erweisen sich oft als nicht hinreichend stabil. Darüber hinaus führt die Keimabtötung in der Regel zu unerwünschten Ablagerungen auf den Oberflächen, die die weitere bakterizide Wirkung maskieren und die Grundlage für eine nachfolgende Bakterienbesiedelung bilden.

W. Kohnen et al. berichten in ZBl. Bakt. Suppl. 26, Gustav Fischer Verlag, Stuttgart-Jena-New York, 1994, Seiten 408 bis 410, daß die Adhäsion von Staphylococcus epidermidis auf einem Polyurethanfilm vermindert wird, wenn der Film durch eine Glimmentladung in Gegenwart von Sauerstoff vorbehandelt und dann mit Acrylsäure gepfropft wird.

Bei Gegenständen zur Verwendung für medizinische Zwecke. d.h. für Untersuchungen. Behandlungen und Eingriffe, wie zuvor beschrieben. spielen nicht nur die bakterienabweisenden Eigenschaften eine Rolle, vielmehr kommt es auch auf Blutverträglichkeit, d.h. auf eine möglichst lange Blutgerinnungszeit bzw. möglichst wenig ausgeprägte thrombogene Eigenschaften an. Nach der internationalen Patentanmeldung WO 94/17904 lassen sich Membranen für medizinische Zwecke durch Behandlung mit einem Niederdruckplasma so modifizieren, daß u.a. ihre thrombogenen Eigenschaften gegenüber unbehandelten Membranen herabgesetzt sind. Unter den geeigneten Plasma-bildenden Gasen wird auch Schwefeldioxid aufgeführt. J.-C. Lin et al. beschreiben in Biomaterials **16** (1995), 1017-1023, die Plasma-Behandlung der inneren Oberfläche von LDPE-Rohren, wobei wiederum Schwefeldioxid als plasmabildendes Gas eingesetzt werden kann. Die Autoren berichten, daß die durch SO₂-Plasma modifizierten Oberflächen Sulfonatgruppen enthielten und stark hydrophil, aber in höherem Maße thrombogen waren als die unbehandelten Oberflächen. Die Autoren vermuten, daß dies auf die kombinierte Wirkung von Oberflächenchemie, also der Sulfonierung, und der Hydrophilie der Oberflächen zurückgeht.

Der vorliegenden Erfindung lag die Aufgabe zugrunde, ein nachhaltig blutverträgliches und bakterienabweisendes Blockcopolymer bereitzustellen, das ohne Wirkungsverlust auf der Oberfläche von Substraten, insbesondere von Kunststoffsubstraten dauerhaft fixiert werden kann und keine Inaktivierung durch abgetötete Bakterien oder sonstige Ablagerungen zeigt.

### 2. Kurzbeschreibung der Erfindung

Es wurde überraschenderweise gefunden, daß diese Aufgabe gelöst wird durch ein Blockcopolymer, das Sulfonat- und Carboxylgruppen tragende Blöcke A sowie Polyurethan- oder Polyesterblöcke B enthält.

Das molare Verhältnis von Carboxylgruppen zu Sulfonatgruppen, die zusammenwirkend die erwünschten blutverträglichen und bakterienabweisenden Eigenschaften der Blockcopolymeren ergeben, beträgt zweckmäßig 0,1 bis 10, vorteilhaft 0,2 bis 10, insbesondere 0,2 bis 5.

Ein anderer Gegenstand der Erfindung ist ein Verfahren zur Herstellung dieses Blockcopolymers, bei dem man ein Copolymer C, das Hydroxylgruppen und/oder Aminogruppen sowie mindestens ein sulfonatgruppenhaltiges Monomer und mindestens ein carboxylgruppenhaltiges Monomer enthält, mit einem Polyurethan D mit endständigen Isocyanatgruppen oder einem Polyester D mit endständigen Carbonsäurechloridgruppen umsetzt.

Bei einer besonderen Variante dieses Verfahrens wird das Polyurethan D in situ, also in Gegenwart des Copolymers C erzeugt und mit diesem umgesetzt.

Ein weiterer Gegenstand der Erfindung ist ein Verfahren zur Beschichtung von Polymersubstraten, bei dem man das Blockcopolymer auf deren Oberfläche aufbringt.

Bei einer besonderen Variante dieses Verfahrens wird das Blockcopolymer auf ein Polyurethansubstrat aufgebracht, sofern die Blöcke B Polyurethanblöcke sind, und auf Polyestersubstrate, wenn die Blöcke B Polyesterblöcke sind.

Ein anderer Gegenstand der Erfindung sind Compounds aus mindestens einem der erfindungsgemäßen Blockcopolymeren und mindestens einem anderen Polymeren. Vorteilhaft ist das andere Polymer ein Polyurethan, wenn die Blöcke B Polyurethanblöcke sind, und ein Polyester, wenn die Blöcke B Polyesterblöcke sind.

Schließlich sind ein Gegenstand der Erfindung Erzeugnisse, die ganz oder teilweise mit den erfindungsgemäßen Blockcopolymeren beschichtet sind oder ganz oder teilweise aus einem erfindungsgemäßen Compound bestehen und sich zur Verwendung für hygienische, technische, nahrungsmittel- und biotechnische sowie insbesondere für medizinische Zwecke eignen.

### 3. Vorteile der Erfindung

Das erfindungsgemäße Blockcopolymer und Beschichtungen oder Compounds daraus sind über lange Zeit gut blutverträglich und wirken heparinartig. Gleichzeitig vermindern sie die Adhäsion und Vermehrung von Bakterien in einem hohen Maße auch über lange Zeit. Von dieser Wirkung betroffene Bakterien sind u.a. Staphylococcus aureus, Staphylococcus epidermidis, Streptococcus pyogenes, Klebsiella pneumoniae, Pseudomonas aeruginosa und Escherichia coli. Wenn es insbesondere bei medizinischen Verwendungen darauf ankommt, daß das Blockcopolymer bzw. ein Beschichtung daraus frei von migrationsfähigen Monomeren oder Oligomeren ist, kann man diese mit geeigneten Lösemitteln, wie Ethanol, extrahieren. Die physikalischen und chemischen Eigenschaften des Substratmaterials bleiben nach der Beschichtung praktisch unverändert. Unerwünschte Nebenwirkungen durch freigesetzte körperfremde Stoffe oder durch abgetötete Bakterien treten nicht ein.

### 4. Detaillierte Beschreibung der Erfindung

### 4.1 Das Copolymer C und die Blöcke A

Aus dem Copolymer C des Herstellverfahrens werden die Blöcke A des Blockcopolymers. Die Aussagen zum Copolymer C gelten daher mutatis mutandis auch für die Blöcke A, und vice versa. Aufgrund ihrer Entstehung durch eine radikalisch initiierte Polymerisationsreaktion haben die Copolymeren C ein Kohlenstoffgerüst. Von den verschiedenen Copolymeren C, die Hydroxyl- und/oder Aminogruppen (zur Verknüpfung mit dem Polyurethan- oder Polyester B) sowie mindestens ein sulfonatgruppenhaltiges Monomer und mindestens ein carboxylgruppenhaltiges Monomer enthalten, werden in der Folge die Copolymeren C1, C2 und C3 näher beschrieben.

### 4.1.1 Das Copolymer C1

Das Copolymer C1 ist aus den Monomeren (a), (b) und (c) aufgebaut, die olefinisch ungesättigt und in einer radikalisch initiierten Reaktion zu einem Terpolymer polymerisierbar sind.

Das **Monomer (a)** ist ein sulfonatgruppenhaltiges Monomer. Als Beispiele seien Vinylsulfonate und Styrolsulfonate genannt, wie Natriumvinylsulfonat und insbesondere Natriumstyrolsulfonat (o- und p-Isomere), sowie Natriummethallylsulfonat, Man kann auch die entsprechenden olefinisch ungesättigten Sulfonsäuren als Monomere für die Copolymerisation einsetzen und erhält dann ein Copolymer mit Sulfonsäuregruppen. Solche Copolymere bzw. daraus hergestellte Blockcopolymere gehören auch zum Bereich der vorliegenden Erfindung. Wenn man die Sulfonsäuregruppen nachträglich neutralisiert, z.B. mit Natriumhydroxid, erhält man Produkte, die den von vornherein mit olefinisch ungesättigten Sulfonatmonomeren hergestellten entsprechen.

Das **Monomer (b)** ist ein carboxylgruppenhaltiges Monomer. Beispiele hierfür sind u.a. Acrylsäure, Methacrylsäure, 4-Vinylsalicylsäure, Vinylessigsäure, Zimtsäure, 4-Vinylbenzoesäure, 2-Vinylbenzoesäure, Crotonsäure, Isocrotonsäure, Methylmaleinsäure, Dihydroxymaleinsäure, Fumarsäure, Methylfumarsäure, Dimethylfumarsäure, Allylessigsäure und insbesondere Maleinsäure. Die für die Polymerisation eingesetzten Monomeren können anstelle der Carboxylgruppen zunächst davon abgeleitete Gruppen enthalten, z.B. Ester-, Amid-, Nitril- oder Anhydridgruppen, die nach der Polymerisation in üblicher Weise in Carboxylgruppen umgewandelt werden können. Weiterhin kann die Carboxylgruppe im Terpolymer auch ganz oder teilweise in Form von Carboxylatgruppen vorliegen. Dies kann man durch Neutralisieren der Carboxylgruppen erreichen, z.B. durch Natriumhydroxid, oder aber dadurch, daß man bei der Copolymerisation anstelle der oder neben den erwähnten carboxylgruppenhaltigen Monomeren die entsprechenden carboxylatgruppenhaltigen Verbindungen einsetzt, Solche carboxylatgruppenhaltigen Copolymere bzw. die daraus resultierenden Blockcopolymere gehören zum Bereich der vorliegenden Erfindung.

Das **Monomer (c)** ist ein hydroxyl- oder aminogruppenhaltiges Monomer. Zu den hydroxylgruppenhaltigen Monomeren gehören u.a. Hydroxyethylacrylat, das besonders bevorzugte Hydroxyethylmethacrylat (HEMA) sowie 4-Hydroxybutylacrylat und 4-Hydroxybutylmethacrylat. Ein geeignetes aminogruppenhaltiges Monomer ist z.B. 4-Aminostyrol.

Natürlich kann man anstelle eines einzelnen Monomers (a), (b) oder (c) entsprechende Mischungen einsetzen, z.B. statt Natrium-p-styrolsulfonat allein eine Mischung aus Natrium-p-styrolsulfonat und Natrium-p-vinylsulfonat. Weiterhin können die genannten Monomeren außer der olefinischen Doppelbindung und der Sulfonat- bzw. Carboxylgruppe weitere funktionelle Gruppen enthalten, sofern sie die Reaktionen nicht stören und die gewünschten biologischen Wirkungen nicht beeinträchtigen.

Das Copolymer C1 kann ausschließlich aus den Monomeren (a), (b) und (c) bestehen. In manchen Fällen ist es möglich und zweckmäßig, zur Modifizierung der biologischen und/oder der Verarbeitungseigenschaften des erfindungsgemäßen Blockcopolymers bei der Herstellung des Copolymers C1 untergeordnete Mengen, z.B. bis zu 30 Molprozent, bezogen auf die Summe der Monomeren (a), (b) und (c), von weiteren Monomeren (d) mit anderer oder ohne Funktionalität mitzuverwenden. Als Beispiele für solche Monomere seien Vinylketone, wie Vinylmethylketon oder Vinylbutylketon; vinylaromatische Monomere, wie Styrol, α-Methylstyrol oder Vinyltoluol; Vinylester, wie Vinylacetat oder Vinylpropionat; Olefine, wie Ethylen, Propylen, 1-Buten und 1-Octen; Diolefine, wie 1,3-Butadien und Isopren; sowie N-Vinylpyrrolidon genannt. Blockcopolymere mit geringen Mengenanteilen solcher Monomere sind ebenfalls erfindungsgemäße Blockcopolymere.

Die Monomeren (a), (b), (c) und gegebenenfalls (d) können im Copolymer C1, je nach Reaktionsführung bei der Polymerisation, als Blöcke oder statistisch verteilt vorliegen. Das Copolymer C1 wird in üblicher Weise durch radikalisch initiierte Polymerisation der Monomeren (a), (b), (c) und gegebenenfalls (d) hergestellt, vorteilhaft durch Lösungs- oder Emulsionspolymerisation. Ein gut geeignetes Lösemittel ist Wasser, in dem die Monomeren und auch das Copolymer zumeist gut löslich sind. Gut geeignet sind auch stark polare organische Lösemittel, wie Dimethylformamid, Dimethylacetamid und Dimethylsulfoxid. Zu den anderen geeigneten Lösemitteln zählen Tetrahydrofuran und Aceton. Für eine gegebene Kombination von Monomeren läßt sich ein geeignetes Lösemittel durch orientierende Versuche unschwer ermitteln.

Geeignete Polymerisationsinitiatoren sind u.a. Azonitrile, Alkylperoxide, Acylperoxide, Hydroperoxide, Peroxyketone, Peroxyester und Percarbonate sowie alle üblichen Photoinitiatoren.

Die Polymerisation wird thermisch, z.B. durch Erhitzen auf 60 bis 100°C, oder durch Strahlung mit entsprechender Wellenlänge initiiert. Man kann z.B. einen Teil der Monomeren (a), (b) und (c) sowie gegebenenfalls (d) vorlegen, die Polymerisation starten und die restlichen Monomeren als Gemisch in den Reaktor einführen, wobei man durch Kühlen zweckmäßig eine Temperatur von 60 bis 100°C aufrechterhält. Auf diese Weise erhält man ein statistisches Copolymer C1. Wenn man ein bestimmtes Monomer oder einen Anteil davon vorlegt und die anderen Monomeren sowie gegebenenfalls die Restmenge des vorgelegten Monomers jeweils für sich portionsweise zugibt, entsteht ein Blockcopolymer C1. Natürlich sind diese Varianten idealtypische Grenzfälle. In der Praxis erhält man schon infolge der unterschiedlichen Reaktionsgeschwindigkeit der verschiedenen Monomeren (i), (ii) und (iii) neben Bereichen mit weitgehend statistischer Verteilung auch solche mit überwiegender Blockstruktur und im zweiten Falle neben den Blöcken auch Übergangszonen mit statistischer Verteilung der Bausteine.

Nach Beendigung der Polymerisation erhält man, je nach den Anteilen der Monomeren (a), (b) und (c) sowie gegebenenfalls (d) und dem verwendeten Lösemittel, eine Lösung oder Emulsion des Copolymers C1 mit Feststoffgehalten, die z.B. bei Lösungspolymerisation 5 bis 20 Gew.-% und bei Emulsionspolymerisation 40 bis 60 Gew.-% betragen können,

Das Copolymer C1 und dessen Herstellung sind Gegenstand der gleichzeitig anhängigen Patentanmeldung 197 26 738.6.

### 4.1.2 Das Copolymer C2

Das Copolymer C2 wird in einer zweistufigen Reaktion hergestellt. Zunächst wird aus den Monomeren (a) und (b) ein primäres Copolymer hergestellt, das nach der Polymerisation modifiziert wird, wie in der Folge erläutert, Die im Zusammenhang mit dem Copolymer C1 gegebenen Erläuterungen zu den Monomeren (a) und (b), zu gegebenenfalls darin enthaltenen weiteren funktionellen Gruppen, zu gegebenenfalls mitzuverwendenden weiteren Monomeren (d) mit anderer oder ohne Funktionalität sowie zum Polymerisationsverfahren gelten entsprechend.

Nach der Polymerisation wird das primäre Copolymer in einem zweiten Reaktionsschritt in einer polymeranalogen Reaktion mit mindestens einer polyfunktionellen NCO- und COCl-reaktiven Verbindung zum Copolymer C2 umgesetzt, Dadurch wird mindestens eine Art von NCO- und COCl-reaktiven Gruppen in das primäre Copolymer eingeführt, zu denen insbesondere Hydroxyl- und Aminogruppen gehören. Als polyfunktionelle NCO- und COCl-reaktive Verbindungen eignen sich vorzugsweise Polyole, wie Diole oder Triole: Aminoalkohole, wie Amino- oder N-Alkylaminoalkanole: sowie Polyamine, wie Diamine und Triamine. Als Beispiele seien genannt: Ethylenglykol, Propylenglykol, 1,4-Butandiol, 1.6-Hexandiol, Trimethylolpropan, Ethanolamin, N-Methylethanolamin, Ethylendiamin, Propylendiamin, Hexamethylendiamin und Diethylentriamin. Als Ergebnis der Umsetzung erhält man NCO- und COCl-modifiziertes primäres Copolymer, in dem die NCO- und COCl-reaktiven Gruppen in Seitenketten angeordnet sind, welche über Carbonester- oder Carbonamidgruppen an das aus Kohlenstoffatomen bestehende Gerüst des primären Copolymers gebunden sind.

Zur NCO- und COCl-reaktiven Modifizierung kann das primäre Copolymer in einem Autoklaven mit einem Unterschuß, bezogen auf die Carboxylgruppen des Copolymers, an Aminoalkohol, Polyamin oder Polyol bei erhöhter Temperatur und unter erhöhtem Druck umgesetzt werden. Der Verlauf der Umsetzung kann mittels potentiometrischer Titration oder NMR-Spektroskopie verfolgt werden. Alternativ kann die Modifizierung auch drucklos vorgenommen werden, indem man das Copolymer und den modifizierenden Stoff in einem hochsiedenden Lösemittel erhitzt, zweckmäßig in Gegenwart eines Schleppmittels für das entstehende Wasser.

Da bei der Umsetzung des primären Copolymers mit der polyfunktionellen NCO- und COCl-reaktiven Verbindung zum Copolymer C2 Carboxylgruppen verbraucht werden, muß der molare Anteil der Carboxylgruppen an der Gesamtfunktionalität des primären Copolymers (d.h. Sulfonatplus Carboxylgruppen) entsprechend höher sein, wenn ein bestimmtes Verhältnis von Carboxyl- zu Sulfonatgruppen für das Copolymer C2 (und damit für das erfindungsgemäße Blockcopolymer) vorgesehen ist. Die Menge der bei dieser Umsetzung eingesetzten polyfunktionellen NCO- und COCl-reaktiven Verbindung hängt von deren Funktionalität (bi-, trifunktionell), dem molaren Anteil der Carboxylgruppen im primären Copolymer und dem gewünschten molaren Verhältnis von Carboxyl- zu Sulfonatgruppen im Copolymer C2 bzw. im erfindungsgemäßen Blockcopolymer ab.

Das Copolymer C2 und dessen Herstellung sind Gegenstand der gleichzeitig anhängigen Patentanmeldung 197 26 737.8.

### 4.1.3 Das Copolymer C3

Ein Copolymer C3 ist dadurch erhältlich, daß man ein Copolymer C1 in einer polymeranalogen Reaktion, wie sie zur Herstellung des Copolymers C2 verwendet wird, mit mindestens einer polyfunktionellen NCO- und COCl-reaktiven Verbindung zum Copolymer C3 umsetzt. Dadurch werden zusätzlich zu den im Copolymer C1 bereits vorhandenen NCO- und COCl-reaktiven Gruppen weitere NCO- und COCl-reaktiven Gruppen eingeführt Die Erläuterungen im Zusammenhang mit den Copolymeren C1 und C2 gelten entsprechend.

Das Copolymer C3 und dessen Herstellung sind Gegenstand der gleichzeitig anhängigen Patentanmeldung 197 26 738.6.

### 4.2. Polyurethan oder Polyester D und Polyurethan- oder Polyesterblöcke B

Die Polyurethane oder Polyester D stehen in demselben Verhältnis zu den Polyurethan- oder Polyesterblöcken B wie die Copolymeren C zu den Sulfonat- und Carboxylgruppen tragenden Blöcken A. Die Erläuterungen zu D gelten also sinngemäß auch für B. Aufgrund ihrer Herstellungsweise haben die Polyurethane und Polyester D sowie die Blöcke B kein reines Kohlenstoffgerüst, sondern ein Kohlenstoffgerüst mit eingebauten Urethan- bzw. Carbonesterbrücken.

### 4.2.1 Polyurethan D mit endständigen Isocyanatgruppen und Polyurethanblock B

Die Polyurethane D werden durch Umsetzung von Polyisocyanaten mit Polyolen hergestellt, wobei ein Äquivalent-Überschuß an NCO-Gruppen gegenüber den OH-Gruppen angewandt wird, dessen Ausmaß das durchschnittliche Molgewicht bestimmt. Dieses darf nicht zu hoch und die Polyurethane dürfen allenfalls schwach vernetzt sein, damit die Produkte flüssig sind und in protischen oder aprotischen Lösemitteln löslich bleiben.

Geeignete Polyisocyanate sind u.a. Toluylendiisocyanat (TDI), Diphenylmethandiisocyanat (MDI), Dicyclohexylmethandiisocyanat (H-MDI), Hexamethylendiisocyanat (HDI) und/oder Isophorondiisocyanat (IPDI). Geeignete Polyole sind z.B. Ethylenglykol, Propylenglykol, 1,4-Butandiol, 1,6-Hexandiol, Neopentylglykol sowie langkettige Diole, wie Polyethylenglykol 600, Polyethylenglykol 1000 sowie entsprechende Polypropylenglykole und Poly(tetramethylenoxy)glykole. Solche langkettigen Polyalkylenglykole sind unter der Bezeichnung Hypol^{(R)} von Hampshire Chemical GmbH, Heidelberg, und unter den Bezeichnungen Vestanat^{(R)} bzw. Vesticoat^{(R)} von Hüls AG erhältlich, Triole und Tetraole, wie Trimethylolpropan und Pentaerythrit, sind wegen ihrer vernetzenden Eigenschaften nur in geringen Mengen zusammen mit Diolen einsetzbar. Die Herstellung der Polyurethane mit endständigen Isocyanatgruppen ist in der Polyurethanchemie gut bekannt und braucht daher nicht weiter erläutert zu werden.

Als Polyurethane D werden auch solche Polymere angesehen, deren Bausteine zwar zu mindestens 50 % durch Urethanbrücken verknüpft sind, die aber daneben auch nach anderen Verknüpfungsprinzipien aufgebaut sind, z.B. Harnstoff- oder Biuretbrücken enthalten.

### 4.2.2 Polyester mit endständigen Carbonsäurechloridgruppen D und Polyesterblöcke B

Die Polyester D mit endständigen Carbonsäurechloridgruppen werden in zwei Stufen hergestellt. Zunächst stellt man durch Polykondensation von Polycarbonsäuren und Polyolen einen Polyester her, wobei ein Äquivalent-Überschuß an Carboxylgruppen gegenüber den Hydroxylgruppen angewandt wird, dessen Ausmaß das durchschnittliche Molgewicht bestimmt. Die Herstellung von Polyestern aus Polycarbonsäuren und Polyolen, zweckmäßig in Gegenwart saurer Katalysatoren und unter Entfernung des Reaktionswassers, ist eine bekannte Reaktion, die hier nicht weiter erläutert zu werden braucht. Anschließend werden die überschüssigen, endständigen Carboxylgruppen in üblicher Weise, z.B. mit Phosgen, Thionylchlorid, Phosphortrichlorid, Phosphorpentachlorid oder besonders schonend mit Oxalyldichlorid, in Carbonsäurechloridgruppen umgewandelt. Das Molgewicht der Polyester D darf wiederum nicht zu hoch und die Polyester D dürfen wiederum nicht zu stark verzweigt sein, damit die Produkte flüssig sind und in protischen oder aprotischen Lösemitteln löslich bleiben.

Für die Herstellung der Polyester D geeignete Polycarbonsäuren sind u.a. Adipinsäure, Dodecan-1,10-dicarbonsäure, Naphthalin-1,5-dicarbonsäure sowie Phthalsäure, Isophthalsäure und/oder Terephthalsäure. Geeignete Polyole sind z.B. die unter 4.2 zuvor genannten.

Als Polyester D werden auch solche Polymere angesehen, deren Bausteine zwar zu mindestens 50 % durch Esterbrücken verknüpft sind, die aber daneben auch nach anderen Verknüpfungsprinzipien aufgebaut sind, z.B. Amid-, Imid- oder Etherbrücken enthalten.

### 4.4 Herstellung der Blockcopolymeren

Die Blockcopolymeren werden durch Umsetzung der hydroxyl- und/oder aminogruppenhaltigen Copolymeren C1, C2 oder C3 mit den beschriebenen Polyurethanen oder Polyestern D gewonnen. Bei der Umsetzung mit einem Polyurethan entstehen aus dessen endständigen Isocyanatgruppen in einer Polyadditionsreaktion die Blöcke verknüpfende Urethan- oder Harnstoffbrücken. Bei der Umsetzung mit Polyestern mit endständigen Carbonsäurechloridgruppen entstehen in einer Polykondensationsreaktion unter Freisetzung von Chlorwasserstoff die Blöcke verknüpfende Carbonester- oder Carbonamidbrücken.

Die Polyadditionsreaktion verläuft schon unter milden Bedingungen praktisch vollständig, Sie entspricht der aus der Polyurethanchemie gut bekannten Umsetzung eines NCO-haltigen Präpolymers mit einem kettenverlängernden Polyol und braucht daher ebenfalls nicht im einzelnen beschrieben zu werden. Man startet z.B. die Reaktion durch Mischen eines Teiles der beiden Komponenten und fügt nach und nach die restlichen Anteile zu, wobei man durch Außen- oder Innenkühlung dafür sorgt, daß die Reaktion nicht zu heftig wird. Die Reaktionstemperatur liegt in der Regel unterhalb von 100°C. Bei höherviskosen Polyurethanen kann man die Viskosität herabsetzen und das Durchmischen der Komponenten fördern, indem man ein inertes Lösemittel zusetzt, das nach Beendigung der Reaktion entfernt werden oder, sofern es die weitere Verwendung des Blockcopolymers nicht stört, im Reaktionsgemisch verbleiben kann.

Bei einer Variante der Polyadditionsreaktion, die zu Blockcopolymeren mit Polyurethanblöcken B führt, setzt man das Copolymer C1, C2 oder C3 nicht mit einem vorgebildeten Polyurethan um, sondern stellt es in situ, also in Gegenwart des Copolymers aus einem Polyisocyanat und einem Polyol her und setzt es mit diesem im Reaktionsgemisch um. Bei gleichen Äquivalentverhältnissen von Isocyanatgruppen zu den gesamten Hydroxyl - oder Aminogruppen im Copolymer C1, C2 oder C3 sowie im Polyol entstehen ähnliche Blockcopolymere.

Auch die erwähnte Polykondensationsreaktion, die Blockcopolymere mit Polyesterblöcken B ergibt, ist für sich gut bekannt und bedarf keiner weiteren Erläuterung. Sie verläuft schon bei Raumtemperatur und unter Wärmeentwicklung, so daß man zweckmäßig durch Kühlung die gewünschte Temperatur einhält. Vorteilhaft erhitzt man das Reaktionsgemisch nach dem Abklingen der exothermen Reaktion noch einige Zeit, z.B. 2 Stunden, auf höhere Temperaturen, z.B. auf 100°C. Besonders glatt und schonend verläuft die Umsetzung, wenn man zur Neutralisation des entstehenden Chlorwasserstoffs eine Base zusetzt, z.B. ein tertiäres Amin, wie Triethylamin.

### 4.5 Verwendung der erfindungsgemäßen Blockcopolymeren

Die erfindungsgemäßen Blockcopolymeren eignen sich wegen ihrer erwähnten bioaktiven Eigenschaften als Beschichtungsmittel in einem Verfahren zur Beschichtung von Polymersubstraten, insbesondere von solchen, die mit den Blockcopolymeren verträglich sind. Das ist dann der Fall, wenn identische oder ähnliche repetierende Einheiten in erheblichem Umfang sowohl im Blockcopolymer als auch im Polymersubstrat vorkommen. Besonders bevorzugte Polymersubstrate sind Polyurethane, wenn die Blöcke B im erfindungsgemäßen Blockcopolymer Polyurethanblöcke sind, und Polyester, wenn es sich bei den Blöcken B um Polyesterblöcke handelt. Offenbar bewirkt die chemische Verwandtschaft der Blöcke und der Substrate eine besonders gute Haftung. Möglicherweise findet beim Erhitzen des beschichteten Substrats auf eine Temperatur nahe dem Schmelz- oder Erweichungspunkt der niedriger schmelzenden oder erweichende Komponente (Blockcopolymer oder Substrat) ein haftungsförderndes Anlösen oder Ineinanderlösen im Grenzflächenbereich statt.

Zur Beschichtung der Polymersubstrate wird das erfindungsgemäße Blockcopolymer in einem inerten Lösemittel, z.B. in Tetrahydrofuran. Aceton oder Dimethylformamid, gelöst und die Lösung auf bekannte Weise, z.B. durch Tauchen, Spritzen, Rakeln, Streichen oder Spin-Coaten, auf das Substrat aufgebracht.

Bei einer Variante des Beschichtungsverfahrens beschichtet man das Polymersubstrat nicht mit dem Blockcopolymer, sondern mit einer Mischung aus einem Copolymer C1, C2 oder C3 einem blockierten Polyisocyanat und einem Polyol. Geeignete Blockierungsmittel sind z.B. Methylethylketoxim und ε-Caprolactam. Beim Erhitzen des beschichteten Substrats auf Temperaturen oberhalb von 100 bis 140°C wird das Blockierungsmittel abgespalten, und das Polyisocyanat reagiert mit dem Copolymer C1, C2 oder C3 und dem Polyol zu dem erfindungsgemäßen Blockcopolymer, wie zuvor beschrieben.

Eine andere Verwendung der erfindungsgemäßen Blockcopolymeren ist die zur Herstellung von Compounds mit anderen Polymeren, insbesondere mit solchen Polymeren, die mit den jeweiligen Blockcopolymeren verträglich sind. Das ist wiederum dann der Fall, wenn identische oder ähnliche repetierende Einheiten in erheblichem Umfang sowohl im Blockcopolymer als auch in dem anderen Polymer vorkommen. Besonders bevorzugte Polymere sind Polyurethane, wenn die Blöcke B im erfindungsgemäßen Blockcopolymer Polyurethanblöcke sind, und Polyester, wenn es sich bei den Blöcken B um Polyesterblöcke handelt. Infolge der chemischen Verwandtschaft der Blöcke und der Polymeren lassen sich die Komponenten in üblichen Mischvorrichtungen, wie Schneckenextrudern oder Zweiwellenextrudern, unschwer mischen und zeigen die Mischungen beim Abkühlen und bei längerer Lagerung keinerlei Entmischungserscheinungen.

Erzeugnisse, die ganz oder teilweise mit den erfindungsgemäßen Blockcopolymeren beschichtet sind oder ganz oder teilweise aus den erwähnten Compounds bestehen, eignen sich für viele Verwendungen, bei denen es darauf ankommt, Bakterienadhäsion und -wachstum zu vermeiden oder zurückzudrängen. Sie sind also u.a. für hygienische, technische, nahrungsmittel- und und biotechnische Zwecke geeignet, wie sie beispielhaft zuvor beschrieben wurden. Die Gegenstände eignen sich vorzugsweise für medizinische Verwendungen, besonders dann, wenn es gleichzeitig auf bakterienabweisende Eigenschaften und Blutverträglichkeit ankommt. Aber auch bei Kontakten mit anderen Körperflüssigkeiten als Blut, wie Lymphe, oder mit Gewebe kommen die vorteilhaften Eigenschaften der erfindungsgemäßen Gegenstände zur Geltung. Medizinische Verwendungen sind z.B. solche als Katheter, z.B. bei der Peritonealdialyse, für Lymphdrainagen, Wundverbände, Tubusse, Stents, Herzklappen, Schläuche, Membranen und Blutbeutel.

Das folgende Beispiel soll die Erfindung weiter erläutern, nicht aber ihren Umfang begrenzen, wie er in den Patentansprüchen dargelegt ist.

### Beispiel

1 g eines NCO-reaktiven Copolymers C2, das durch Aminolyse aus einem primären Copolymer aus Natriumstyrolsulfonat und Maleinsäure hergestellt wurde, wie in Beispiel 2 der gleichzeitig anhängigen Patentanmeldung 197 26 737.8 beschrieben, mit einem Hydroxylgehalt von 0,0019 mol/g sowie 1 g EP-UM 767 (ein lineares Präpolymer auf Basis von Isophorondiisocyanat der Hüls AG mit 0.0017 mol/g endständigen NCO-Gruppen) werden in 100 ml Dimethylformamid (DMF) gelöst. Nach Zusatz von 1,3 mg (3,6 µmol) Di-n-butylzinndilaurat wird das Gemisch 20 min auf 100°C erhitzt. Das Lösemittel wird im Vakuum abdestilliert, und es verbleibt das entstandene Blockcopolymer mit COOH- und SO₃Na-Gruppen, das mit Wasser gewaschen und getrocknet wird.

Das Blockcopolymer wird zu 10 Gew.-% in Tecoflex^{(R)} (ein Polyurethan auf Basis von 4,4'-Methylen(cyclohexylisocyanat), Polytetramethylenoxyglykol und 1,4-Butandiol der Fa. Thermedix, Hamburg, eincompoundiert. Blockcopolymer und Polyurethan mischen sich problemlos und zeigen weder beim Abkühlen noch bei Lagerung über längere Zeiträume Entmischungserscheinungen. Die Messung der primären Bekterienadräsion unter statischen Bedingungen nach der in der gleichzeitig anhängigen Anmeldung 197 26 737.8 gegebenen Vorschrift ergab eine Reduktion von 70% gegenüber Tecoflex^{(R)}.

Das Blockcopolymer wird in eine Tecoflex^{(R)}-Folie eingequollen, indem die Folie 30 min in eine 20 Gew.-% Lösung des Blockcopolymers in DMF gelegt und nach der Entnahme mit Nasser gespült wird. Die Messung der primären Bekterienadhäsion unter statischen Bedingungen nach der in der gleichzeitig anhängigen Anmeldung 197 26 737.8 gegebenen Vorschrift ergab eine Reduktion von 75% gegenüber Tecoflex^{(R)}.

## Patentansprüche

1. Blockcopolymer, enthaltend Sulfonat- und Carboxylgruppen tragende Blöcke A und Polyurethan- oder Polyesterblöcke B.

2. Blockcopolymer nach Anspruch 1, dadurch gekennzeichnet, daß das molare Verhältnis von Carboxylgruppen zu Sulfonatgruppen 0,1 bis 10 beträgt.

3. Blockcopolymer nach Anspruch 1, dadurch gekennzeichnet, daß das molare Verhältnis von Carboxylgruppen zu Sulfonatgruppen 0,2 bis 5 beträgt.

4. Compound, enthaltend mindestens ein Blockcopolymer gemäß Anspruch 1 sowie mindestens ein weiteres Polymer.

5. Compound nach Anspruch 4, dadurch gekennzeichnet, daß das Blockcopolymer und das weitere Polymer gleiche oder ähnliche repetierende Einheiten enthalten.

6. Verfahren zur Herstellung eines Blockcopolymers nach einem der Ansprüche 1 bis 3, dadurch gekennzeichnet, daß man ein Copolymer C, das Hydroxylgruppen und/oder Aminogruppen sowie mindestens ein sulfonatgruppenhaltiges Monomer (a) und mindestens ein carboxlgruppenhaltiges Monomer (b) enthält, mit einem Polyurethan D mit endständigen Isocyanatgruppen oder einem Polyester D mit endständigen Carbonsäurechloridgruppen umsetzt.

7. Verfahren nach Anspruch 6, dadurch gekennzeichnet, daß man das Polyurethan D in Gegenwart des Copolymers C herstellt und im Reaktionsgemisch mit diesem umsetzt.

8. Verfahren nach Anspruch 6, dadurch gekennzeichnet, daß man einen Polyester D verwendet, der in zwei Stufen hergestellt wird, indem man zunächst einen Polyester mit endständigen Carboxylgruppen durch Polykondensation von Polycarbonsäuren mit Polyolen herstellt, wobei ein Äquivalent-Überschuß an Carboxylgruppen gegenüber den Hydroxylgruppen angewandt wird, und anschließend die überschüssigen, endständigen Carboxylgruppen in üblicher Weise mit einem Chlorierungsmittel in Carbonsäurechloridgruppen umwandelt.

9. Verfahren nach einem der Ansprüche 6 bis 8, dadurch gekennzeichnet, daß das Copolymer C ein Copolymer C1 ist, das ein sulfonatgruppenhaltiges Monomer (a), ein carboxylgruppenhaltiges Monomer (b) und ein hydroxyl- und/oder aminogruppenhaltiges Monomer (c) enthält.

10. Verfahren nach einem der Ansprüche 6 bis 8, dadurch gekennzeichnet, daß das Copolymer C ein Copolymer C2 ist, das aus einem primären, ein sulfonatgruppenhaltiges Monomer (a) und ein carboxylgruppenhaltiges Monomer (b) enthaltenden Copolymer durch polymeranaloge Reaktion mit einer polyfunktionellen NCO-reaktiven Verbindung entsteht.

11. Verfahren nach einem der Ansprüche 6 bis 8, dadurch gekennzeichnet, daß das Copolymer C ein Copolymer C3 ist, das aus einem Copolymer C1 durch polymeranaloge Reaktion mit einer polyfunktionellen NCO-reaktiven Verbindung entsteht.

12. Verfahren zur Beschichtung von Kunststoffsubstraten, dadurch gekennzeichnet das das Beschichtungsmittel ein Blockcopolymer nach einem der Ansprüche 1 bis 3 ist.

13. Verfahren nach Anspruch 12, dadurch gekennzeichnet, daß das Kunststoffsubstrat ein Polyurethan ist, sofern die Blöcke B des Blockcopolymers Polyurethanblöcke sind, und ein Polyester, wenn die Blöcke B des Blockcopolymers Polyesterblöcke sind.

14. Verfahren nach Anspruch 12 oder 13, dadurch gekennzeichnet, daß das Beschichtungsmittel durch Tauchen, Spritzen, Rakeln, Streichen oder Spin-Coaten aufgebracht wird.

15. Erzeugnisse, die ganz oder teilweise mit einem Blockcopolymer nach einem der Ansprüche 1 bis 3 beschichtet wurden oder ganz oder teilweise aus einem Compound gemäß den Ansprüche 4 oder 5 bestehen, für hygienische, technische, nahrungsmittel- oder biotechnische Zwecke.

16. Erzeugnisse, die ganz oder teilweise mit einem Blockcopolymer nach einem der Ansprüche 1 bis 3 beschichtet wurden oder ganz oder teilweise aus einem Compound gemäß Anspruch 4 oder 5 bestehen, für medizinische Zwecke.

17. Erzeugnisse nach Anspruch 16, dadurch gekennzeichnet, daß die Erzeugnisse Katheter, Lymphdrainagen, Wundverbände, Tubusse, Stents, Herzklappen, Schläuche, Membranen oder Blutbeutel sind.
